**Europäisches Patentamt**

**European Patent Office**

**Office européen des brevets**

(11) Veröffentlichungsnummer: **0 133 551**
**A2**

# (12) EUROPÄISCHE PATENTANMELDUNG

(21) Anmeldenummer: **84109168.9**

(22) Anmeldetag: **02.08.84**

(51) Int. Cl.⁴: **A 61 K 39/395**
**C 12 N 5/00, G 01 N 33/577**
**A 61 K 37/02, A 61 K 35/14**

(30) Priorität: **16.08.83 DE 3329449**

(43) Veröffentlichungstag der Anmeldung:
**27.02.85 Patentblatt 85/9**

(84) Benannte Vertragsstaaten:
**AT BE CH DE FR GB IT LI LU NL SE**

(71) Anmelder: **Biotest-Serum-Institut GmbH**
**Flughafenstrasse 4**
**D-6000 Frankfurt 71(DE)**

(72) Erfinder: **Sonneborn, Hans H., Dr. Dipl. Biol.**
**Birkeneck 70**
**D-6056 Heusenstamm(DE)**

(72) Erfinder: **Vornhagen, Rolf M., Dr. Dipl. Biol.**
**Nördl. Ringstrasse 19**
**D-6070 Langen(DE)**

(72) Erfinder: **Schwulera, Udo, Dr. Dipl.-Biol.**
**Krebsbachweg 23**
**D-6450 Hanau(DE)**

(74) Vertreter: **Wolff, Hans Joachim, Dr.jur.**
**Dipl.-Chem. et al,**
**Beil, Wolff & Beil Rechtsanwälte Postfach 80 01 40**
**Adelonstrasse 58**
**D-6230 Frankfurt am Main 80(DE)**

(54) Monoklonaler Antikörper, der eine Struktur erkennt die dem Human-Interleukin-2 (TCGF) und der leichten Kette "lambda" von Humanimmunglobulin gemeinsam ist, und Hybridoma-Zell-Linien, die diese monoklonalen Antikörper produzieren.

(57) Monoklonale Antikörper, die eine Struktur erkennen, die dem Human-Interleukin-2 (TCGF) und der leichten Kette λ von Humanimmunglobulin gemeinsam ist und Hybridoma-Zell-Linien, die diesen monoklonalen Antikörper produzieren, lassen sich dadurch herstellen, daß man Tiere, insbesondere Mäuse mit Human-Interleukin-2 (TCGF) immunisiert und die von diesen Tieren erhaltenen Milz-Zellen mit Tier-insbesondere Maus-Myeloma-Zellen unter Bildung eines Hybridomas fusioniert. Die Hybridomas werden als Klone gezüchtet und die von den einzelnen Klonen erhaltenen Antikörper auf ihre Spezifität gegen Human-Interleukin-2 (TCGF) getestet. Klone, die Antikörper produzieren mit einer Spezifität gegen Human-Interleukin-2 (TCGF) werden für die Weiterzüchtung zur Herstellung des Antikörpers selektiert, wobei der Antikörper aus dem Kulturmedium oder den Ascitesflüssigkeiten des Tieres, insbesondere der Maus, die den Hybridomatumor trägt, gewonnen wird.

Unsere Nr. 24 475                    Pr/br

Biotest-Serum-Institut GmbH
Flughafenstraße 4
6000 Frankfürt 71

Monoklonaler Antikörper, der eine Struktur erkennt, die
dem Human-Interleukin-2 (TCGF) und der leichten Kette Λ
von Humanimmunglobulin gemeinsam ist, und Hybridoma-
Zell-Linien, die diese monoklonalén Antikörper
produzieren

Die Erfindung betrifft monoklonale Antikörper, die
eine Struktur erkennen, die dem Human-Interleukin-2
(TCGF) und der leichten Kette Λ von Hummanimmunglobulin gemeinsam ist und Hydridoma-Zell-Linien, die
diese monoklonalen Antikörper produzieren.

In den Ansprüchen und in der Beschreibung werden für
folgende Begriffe folgende Abkürzungen benutzt:

T-Zell-Wuchsfaktor - TCGF
MLC = gemischte Lymphozytenkultur
PEG = Polyethylenglykol
PHA = Phythämagglutinin
SDS = Natriumdodecylsulfat
POD = Peroxidase
Interleukin-2 = IL-2
BJ = Bence Jones Protein

HSA = Humanserumalbumin

MAB = monoklonale Antikörper

CoN A = Concanavalin A

MW = Molekulargewicht

Interleukin-2 (IL-2) (TCGF) ist ein Lymphokin und spielt bei der Regulation der Immunantwort eine entscheidende Rolle.

Dieser zuerst 1976 von Morgan et al., Science 193, 1007 (1976) (1) beschriebene Faktor nimmt eine zentrale Stellung bei der Ausbildung der Immunantwort ein. So wird beispielsweise eine ruhende T-Zelle von einem Antigen sensibilisiert und aktiviert und erhält somit ein Signal. Das gleiche Antigen stimuliert aber auch Makrophagen und Helfer-T-Zellen, die der aktivierten T-Zelle in Form von Interleukin-2 ein 2. Signal geben, woraufhin die Zelle zu proliferieren beginnt.

Die Gewinnung von rohem Human-Interleukin-2 wird von einer Reihe von Autoren beschrieben, u.a. von Ruscetti FW, Gallo RC: Regulation of the production and release of human T cell growth factor, J. Supramol Biol. 13: 1980 (2);
Lindsay P., Schwulera U. und Sonneborn H.H., The Species Specificity of Interleukin-2, 3. internationaler Lymphokin-Kongress, 14.-17. Oktober 1981, Dallas, Texas, (3).

Ferner sind verschiedene Reinigungsmethoden bekannt, die u. a. in DE-OS 31 49 360 (4) beschrieben werden.

Eine Zusammenfassung von Verfahren zur Herstellung, Reinigung und Eigenschaften von IL-2 ist dem Biotest Bulletin 3: 222-227 (1982) (5) zu entnehmen.

Da bisher nur aufwendige biologische Tests zur Messung der IL-2 Aktivität zur Verfügung stehen wäre die Bestimmung von IL-2 mit Hilfe eines monoklonalen Antikörpers von großer Bedeutung; zum einen, um das Protein zu identifizieren und zum anderen, um einen einfachen und reproduzierbaren Test zur Verfügung zu haben.

Außerdem böte ein solcher Antikörper die Möglichkeit, Protein zu reinigen (z.B. für ein Therapieprodukt).

In der EP-A1 0 064 401 (6) wird zwar bereits ein monoklonaler Antikörper beschrieben, der die Interleukin-2-Aktivität hemmt. Dieser Antikörper ist jedoch nur in der Lage, Aktivität zu adsorbieren, kann diese jedoch nicht wieder freigeben. Wie dort beschrieben wird, konnte von den Antikörpersäulen keine Aktivität eluiert werden.

In (5) wird die Herstellung von Hybridomas beschrieben, die im ELISA-Test positiv waren und die Wirkung von IL-2 in $^3$H-Thymidin-Inkorporations-Assays hemmten. Eine genauere Beschreibung und Identifizierung von daraus gewonnenen Antikörpern findet sich dort nicht.

Der Erfindung liegt die Aufgabe zugrunde, eine Möglichkeit zu finden, IL-2 (TCGF) besser zu identifizieren und zu bestimmen, z.B. bei Patienten,und besser zu reinigen und zu charakterisieren.

Diese Aufgabe wird erfindungsgemäß gelöst durch die Bereitstellung einer Hybridoma-Zell-Linie, die einen Antikörper zu produzieren vermag, der eine Struktur erkennt, die dem Human-Interleukin-2 (TCGF) und der leichten Kette $\lambda$ von Humanimmunglobulin gemeinsam ist, bzw. des produzierten Antikörpers.

Überraschenderweise wurde gefunden, daß der erfindungsgemäße Antikörper eine Struktur erkennt, die dem Human-IL-2 (TCGF) und der leichten Kette $\lambda$ von Humanimmunglobulin gemeinsam ist und dadurch genauer definiert werden kann und daß er die gesamte IL-2 Aktivität adsorbiert und davon wieder ein beachtlicher Teil in aktiver Form eluiert werden kann. Damit eignet sich der erfindungsgemäße Antikörper nach Entfernung der leichten Kette $\lambda$ zur hochspezifischen Reinigung von IL-2.

Ein wesentlicher Fortschritt besteht darin, daß sich mit Material, das von einer Antikörpersäule der erfindungsgemäßen monoklonalen Antikörper eluiert wurde, im Vergleich sowohl
mit einer IL-2-Präparation⋎klonierte und nicht-klonierte
T-Zellen        als auch MLC-, PHA und CoN A-Blasten
tierischen und humanen Ursprungs über einen relativ
langen Zeitraum vermehren lassen.

Monoklonale Antikörper lassen sich dadurch erhalten,
daß man ein Tier mit einem Antigen immunisiert, Antikörper
produzierende Zellen von diesem Tier erhält und die Antikörper produzierenden Zellen mit Tumor-Zell-Linien,
z.B. Myeloma-Zell-Linien, fusioniert, wobei man Hybridomas erhält, die isoliert werden und die die monoklonalen Antikörper produzieren (Köhler, G. und Milstein
C., Eur.J.Immunol., 6, 511-519 (7) und Köhler, G. und
Milstein, C., Nature, 256, 495 (1975) (8).

Die Zellen des Hybridomas haben von dem einen "Elternteil" das Vermögen mitbekommen, einen ganz bestimmten
Antikörper zu produzieren. Dem anderen "Elternteil",
dem Myelom, verdanken sie ihre Fähigkeit, sich auf lange
Zeit hin weiter zu teilen.

Diese Hybridomas können entweder in vitro oder aber
als Tumoren in einem Wirtstier gezüchtet werden.

Da jede Antikörper produzierende Zelle (Klon) nur
Antikörper einer einzigen Spezifität bildet, produzieren
die monoklonalen Kulturen der Hybridomas, die aus einem
solchen Klon mit einer Spezifität gezüchtet wurden,
jeweils einen homogenen Antikörper, der entweder aus dem
Kulturmedium von Hybridoma-Kulturen in vitro oder aus
den Zellen, Ascitesflüssigkeiten oder Seren von tumortragenden Wirtstieren erhalten werden kann.

Aufgrund der Homogenität und hohen Spezifität der auf diese Weise hergestellten monoklonalen Antikörper sind letztere äußerst wertvoll für diagnostische Zwecke.

Um die Produktion von Antikörpern mit Spezifität gegen Human IL-2 (TCGF) zu induzieren, werden Tiere mit einer gereinigten IL-2 (TCGF)-Präparation immunisiert. Die dafür ausgewählte Tierspecies ist nicht kritisch, jedoch sollte der Stamm des Tieres genetisch gut definiert sein. Üblicherweise werden dafür Mäuse verwendet, da bei diesen Species verschiedene Inzuchtstämme existieren, die genetisch gut definiert sind, und da verschiedene neoplastische Zellen murinen Ursprungs ebenfalls als gut charakterisierte Kulturen erhältlich sind. Jedoch lassen sich auch andere Tierspezies für diesen Zweck verwenden.

Danach wird eine Milzzellensuspension hergestellt und gewaschen. Die so erhaltenen Antikörper produzierenden Zellen werden mit neoplastischen Zellen fusioniert. Einige Tumorzellen, insbesondere Myeloma-Zellen, lassen sich vorteilhafterweise mit den Antikörper produzierenden Zellen fusionieren und liefern lebensfähige, Antikörper produzierende Kulturen von Hybridomas. Vorzugsweise sollten die Tumorzellen und die Antikörper produzierenden Zellen von der gleichen Species stammen, da dabei die Wahrscheinlichkeit, daß die genetischen und biochemischen Eigenschaften der Elternzellen verträglich sind, größer wird und dadurch lebensfähige Hybridomas produziert werden.

0133551

Es gibt eine Vielzahl gut charakterisierter Myeloma-Zell-Linien, die sich erfindungsgemäß verwenden lassen. Beispiele hierfür sind P3-X63-Ag8, P3-X63-Ag8.653, S 194, Y3, SP2/0, MPC-11 und deren Derivate. Vorzugsweise wird die Linie P3-X63-Ag8.653 (Kearney et al., J.Immunol. 123: 1548 (1979) (9) verwendet, die bei der C.N.C.M. am 6. Oktober 1983 unter der Nr. I-251 hinterlegt wurde. Vorzugsweise wählt man eine Myeloma-Linie aus, die keinen Antikörper produziert, so daß das dabei entstehende Hybridoma nur Antikörperketten von der Eltern-Milz- oder -Lymphknoten-Zelle produziert.

Myeloma-Zellen und Zellen, die von Mäusen erhalten wurden, die mit der Human IL-2 Präparation immunisiert worden waren, werden nach der Methode von Köhler, G. und Milstein, C., (7) fusioniert.

Danach werden die Hybridomas selektiert, wobei jegliches Wachstum die erfolgreiche Hybridisierung von Maus-Milz-Zellen und Maus-Myeloma-Zellen anzeigt.

Klone von Hybridomas können in vitro gezüchtet werden nach den bekannten Gewebekulturmethoden, wie beispielsweise durch Cotton et al., Eur.J.Immunol. 3, 136 (1973) (10) beschrieben. Wahlweise lassen sich die Hybridomas jedoch auch in vivo als Tumoren in einem histokompatiblen Tier oder in athymischen Nacktmäusen züchten. Die Antikörper lassen sich aus dem in vitro Kulturmedium oder aus dem Serum oder der Ascitesflüssigkeit des Tieres nach bekannten Methoden gewinnen.

Die Spezifität des Antikörpers von jedem Klon gegen IL-2 (TCGF) wird mit Hilfe von bekannten

- 8 -

Testmethoden festgestellt, und Klone, die Antikörper
mit der gewünschten Spezifität produzieren, werden
selektiert.

Nachstehendes Beispiel dient der weiteren Erläuterung
der Erfindung.

IL-2 Präparation:

Humane periphere Blutlymphozyten wurden über Ficoll/
Isopaque gereinigt und in RPMI 1640 Medium mit 1 % PHA
48 Stunden bei 37°C und 5 % $CO_2$ inkubiert. Die Zell-
dichte betrug 1 x $10^6$ Zellen/ml.

Der Kulturüberstand wurde zentrifugiert und mit Hilfe einer fraktionierten
Ammoniumsulfatpräzipitation , Gelfiltration über Sephadex G 75 und Dyematrix-blue A Chromatographie gereinigt,
wie in (5) Fig. 7, Fig. 8 und Fig. 6 beschrieben wird.

Dieses Material wurde zur Immunisierung von Mäusen verwendet.

Immunisierung:

BALB/c Mäuse wurden mit 100 µg dieser Präparation, emulgiert in kompletten Freund'schen Adjuvants, i.p. und s.c.
alle 14 Tage immunisiert. Insgesamt erhielten die
Mäuse 5 Injektionen.

Fusion:

4 Tage nach der letzten Boosterinjektion wurden die Mäuse
getötet und die Milzzellen dieser Mäuse mit der Maus-Myeloma
Linie P3-X63-Ag.8.653 entsprechend der in (7) und (8)
beschriebenen Methoden fusioniert.

Dazu wurden die Milzzellen mit den Myelomazellen in einem Verhältnis von 11:1 gemischt und unter Verwendung von PEG 1500 (Roth) fusioniert und in Mikrotiterplatten ausgesät.

Ergebnisse:

Von 333 zeigten 233 Näpfe Wachstum von Klonen.

Die Überstände aus diesen 233 Näpfen wurden im ELISA-Test mit einer mit Humanalbumin (HA) beschichteten und mit einer mit IL-2 Präparation beschichteten Mikrotiterplatte gemessen.

7 Überstände zeigten dabei im ELISA-Test mit der IL-2 Präparation deutlich höhere Extinktionen als mit Humanalbumin (z.B. 0,535 mit IL-2 und 0,225 mit HA), unter ihnen ein Klon der von einer mit BS 50 bezeichneten Hybridoma-Zell-Linie produziert wurde.

Die Hybridoma-Linie BS 50 wurde bei der C .N.C.M. am 5. Juli 1984 unter der Nr. I-313 hinterlegt.

BS 50 wurde mit der limiting dilution Methode kloniert. Von 288 Näpfen zeigten 33 Näpfe Wachstum von nur 1 Klon. Die Überstände wurden wiederum im ELISA getestet und alle 33 Näpfe zeigten im ELISA eine höhere Extinktion als gegen HA.

Ergebnisse mit dem monoklonalen Antikörper gegen PBL human IL-2, der von der Hybridoma-Zell-Linie BS 50 produziert wurde 1.) Hemmung des biologischen IL-2 Tests

Die biologische IL-2 Bestimmung wurde mit MLC oder PHA stimulierten Lymphocyten-Blastenzellen durchgeführt. Dabei war die Menge an eingebautem

- 10 -

$^3$H-Thymidin ein Maß für die IL-2 Aktivität.

Wie aus Tabelle 1 hervorgeht, hemmt der Zusatz des Antikörpers aus BS 50 den Einbau von $^3$H-Thymidin.

Da human IL-2 auch aktiv gegenüber Mauszellen ist, wurde die Hemmung auch in diesem System gemessen.

Der Zusatz von Antikörpern aus BS 50 hemmt auch die $^3$H-Thymidin-Inkorporation in Mausmilz Con A-Blasten-Zellen. (Dies ist neben den ELISA-Daten ein weiterer Hinweis, daß der monoklonale Antikörper gegen IL-2 gerichtet ist.)

2.) ELISA mit verschiedenen Fraktionen nach Gelfiltration von IL-2 Überstand

Die Tabelle 2 zeigt,daß Antikörper aus BS 50 stark mit der Fraktion mit dem Molekulargewicht (MG) 160 000 deutlich mit der Fraktion MG 45 000 und schwach mit der Fraktion 17 000 reagiert.
Sie zeigt auch, daß der monoklonale Antikörper ein Epitop auf dem Protein MG 160 000 und MG 45 000 erkennt, das mindestens auf diesen Molekülen zweimal vorhanden sein muß.

IL-2 hat in der Gelfiltration ein MG von 17 000, während IgG ein Molgewicht von 160 000 hat. Fab hat ein MG von 50 000.
(Ein erster Hinweis, daß der Antikörper aus BS 50 mit IgG reagiert, was auch erklärt, daß das Epitop zweimal vorhanden ist.)

3.) ELISA mit verschiedenen, definierten Proteinen

Zur Abklärung der·Reaktion des Antikörpers ᵛBS 50 mit·IgG wurden
aus·
ELISA-Tests mit definierten· Immunglobulinen und
Teilen von Immunglobulinen (IgG-Fab Teil, IgG-Fc
Teil, leichte Ketten $\lambda$ und $\kappa$ ) durchgeführt.

Der Antikörper aus BS 50 reagiert in diesen Tests deutlich mit
der leichten Kette $\lambda$ und Immunglobulinen, die die leichten
Kette $\lambda$ tragen, wie aus Tabelle 3 ersichtlich ist.

4.) Nachweis der leichten Kette $\lambda$ in der SDS-Elektrophorese mit POD konjugiertem monoklonalem Antikörper   aus BS 50

Bence Jones Proteine vom $\lambda$ - und vom $\kappa$ -Typ wurden
mittels SDS-Elektrophorese getrennt. Der Nachweis
der Proteine erfolgte mit einem blotting Verfahren,
wobei das Protein vom SDS-Gel auf eine Nitrocellulose-
Membran übertragen wurde und dort mit dem Antikörper
im direkten oder indirekten Test nachgeweisen wurde.

Der Nachweis erfolgte in diesem Fall direkt mit POD
konjugiertem Antikörper.

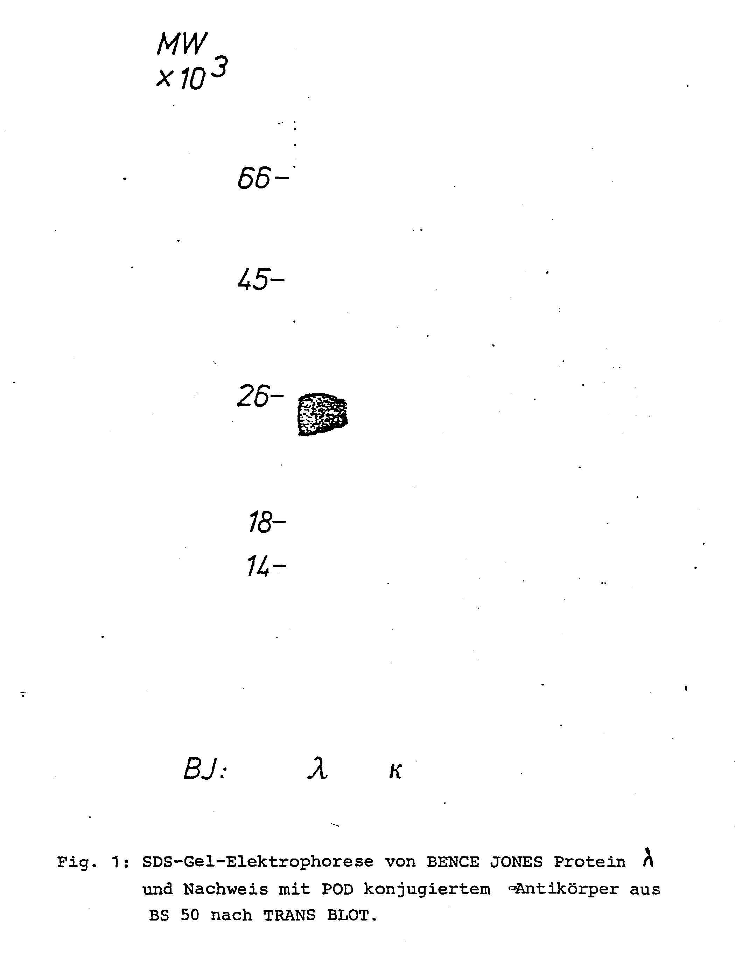

Wie Fig. 1 zeigt, reagiert der Antikörper aus BS 50
deutlich mit der leichten Kette $\lambda$ (MG 25 000).

Die schwache Reaktion mit $\kappa$ ist auf Verunreinigungen
durch $\lambda$ zurückzuführen.

-12-

5.) Bindung von IL-2 an Antikörper aus BS 50-Sepharose Säulen

Nachdem die Reaktion von Antikörpern aus BS 50 mit der leichten Kette $\lambda$ eindeutig gezeigt werden konnte, wurden zur weiteren Überprüfung dieses monoklonalen Antikörpers bezüglich seiner Reaktion mit IL-2 für weitere Versuche $\lambda$-leichte Ketten durch Immundadsorption mit Anti-$\lambda$ Säulen aus IL-2 Überständen entfernt.

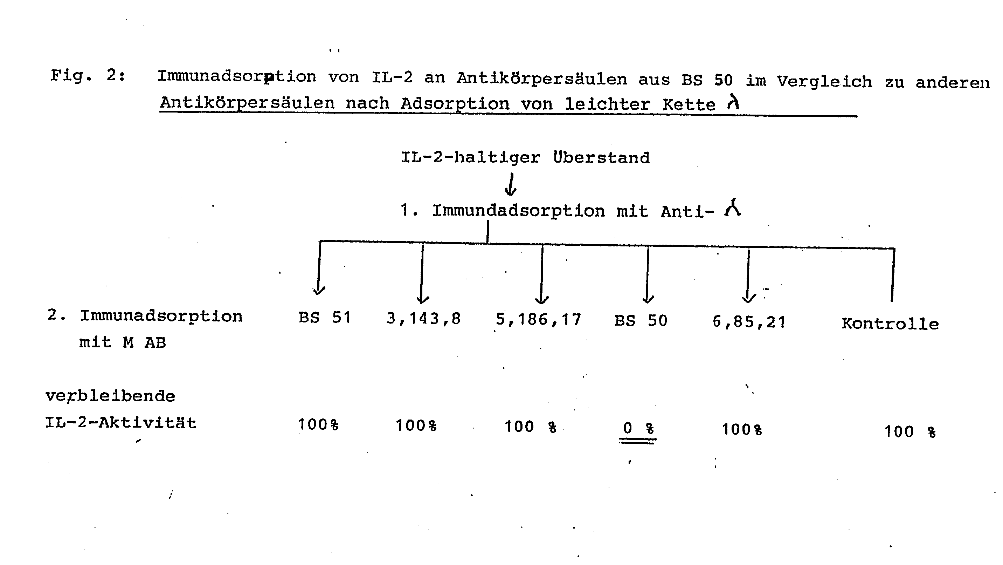

Der auf diese Weise erhaltene $\lambda$-freie IL-2 Überstand wurde dann, wie in Fig. 2 angegeben, in 6 Teilen über 6 verschiedene mit monoklonalen Antikörpern beladene Säulen gegeben, und im Durchfluß wurde die IL-2 Aktivität bestimmt.

Der monoklonale Antikörper aus BS 50 bindet im Vergleich zu den anderen Antikörpern als einziger sämtliche IL-2 Aktivität.

6.) Elution von IL-2 von BS 50 Antikörpersäulen

Wie im vorangegangenen Experiment gezeigt, konnte die gesamte Aktivität adsorbiert werden.

Wie Tabelle 4 zeigt, konnten 27 % der aufgetragenen IL-2 Menge von der BS 50 Säule eluiert werden.

Damit eignet sich der monoklonale Antikörper aus BS 50 zur hochspezifischen Reinigung von IL-2, vorausgesetzt, die leichte Kette $\lambda$ wird vorher entfernt.

7.) Langzeitwuchs von aktivierten T-Zellen mit Antikörpern aus BS 50 eluiertem IL-2

- 13 -

Während die ³H-Thymidin-Inkorporationstests im Prinzip nur Hilfstests sind zur Bestimmung der IL-2 Aktivität, die aber weltweit als IL-2 Test akzeptiert werden, ist der endgültige Nachweis für IL-2 Aktivität die Kultur von aktivierten T-Zellen über einen längeren Zeitraum.

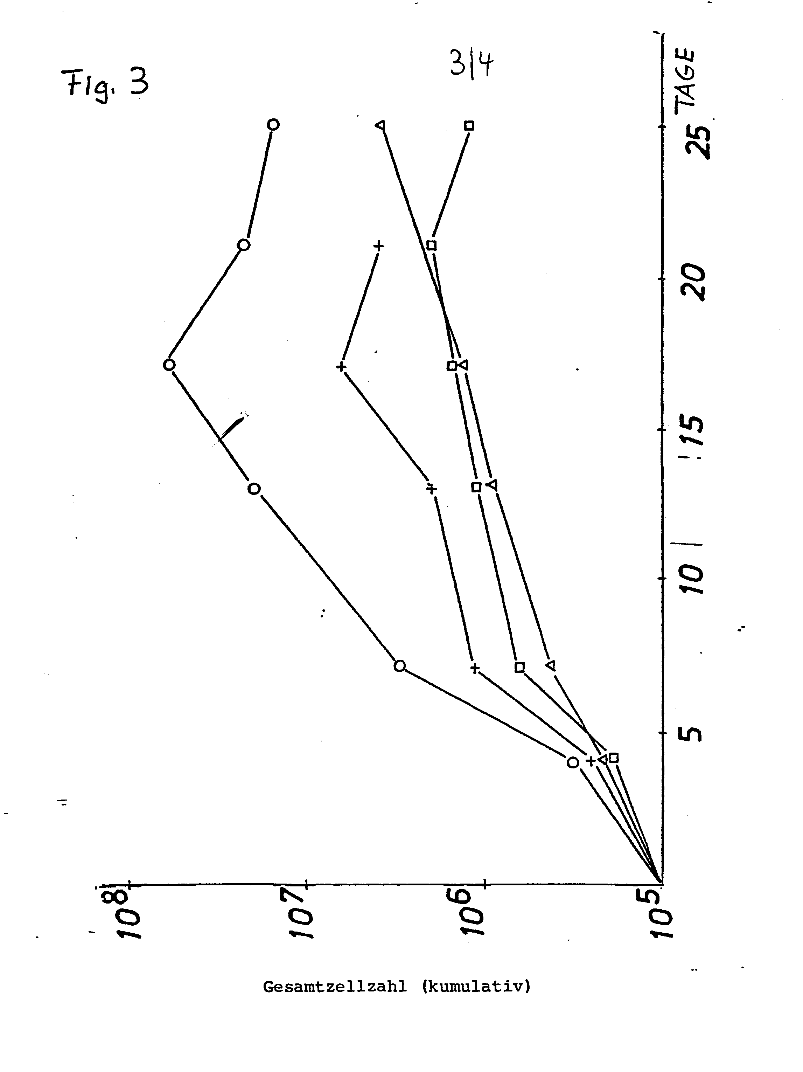

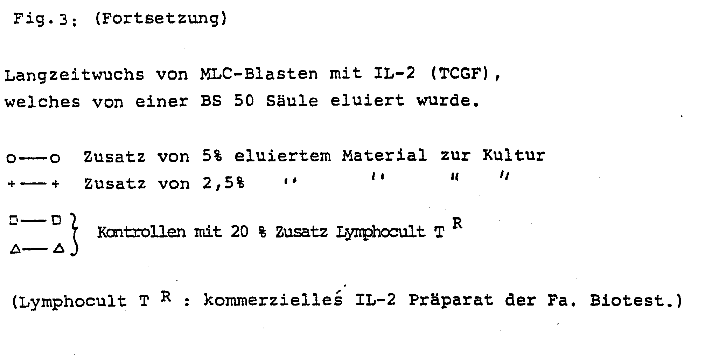

Wie die Fig. 3 zeigt, können mit Material, welches von einer BS 50 Antikörpersäule eluiert wurde, humane MLC-Blasten im Vergleich mit einer IL-2 Präparation über einen Zeitraum von 4 Wochen kultiviert werden.

Der von der Hybridoma-Linie BS 50 produzierte monoklonale Antikörper zeigt somit spezifische Reaktionen mit einem Faktor, der für das in vitro Wachstum von T-Zellen essentiell ist und nach biochemischen Kriterien Human-IL-2 entspricht und mit der leichten Kette λ von Humanimmunglobulin, d.h. der Antikörper erkennt die Struktur (Aminosäurerequenz bzw. Zuckerrest), die dem IL-2 (TCGF) und der leichten Kette λ gemeinsam ist.

## Tabelle 1

Hemmung der $^3$H-Thymidin-Inkorporation durch Klone, die von BS 50 produziert wurden, im IL-2 Test

| Test-Zellen | M AB | Verdünnung von M AB CPM | | | |
|---|---|---|---|---|---|
| | | 1:40 | 1:100 | 1:200 | 1:400 |
| MLC-Blasten- | BS 50 | 449 | 470 | 894 | 22589 |
| Zellen | BS 51 | 544 | 831 | 2644 | 33323 |
| (Human) | Kontroll- | 133567 | 151459 | 211949 | 210028 |
| | medium | 107332 | 131420 | 181913 | 254612 |
| CoN A sti- | BS 50 | 303 | 1042 | 6859 | 35508 |
| umliert | BS 51 | 359 | 603 | 21758 | 28 626 |
| (Mäuse- | Kontroll- | 24462 | 27977 | 33929 | 29539 |
| Milzzellen) | medium | 39952 | 49255 | 27308 | 43140 |

## Tabelle 2

Reaktion von Antikörpern, die von BS 50 produziert wurden, mit drei verschiedenen Fraktionen einer IL-2-Präparation nach Gelchromatographie an Sephadex G 75

ELISA

| Antikörper-<br>Festphase | Antikörper-<br>Flüssigphase | Antigen = IL-2-haltiger Überstand, gereinigt durch Gelfiltration | | |
|---|---|---|---|---|
| | | MW: 160 K | 45 K | 17 K |
| BS 50 | BS 50 POD | +++ | + | +- |
| BS 50 | anderes<br>MAB-POD | - | - | - |

## Tabelle 3

Reaktion der Antikörper aus BS 50 im ELISA-Test mit Immunglobulin-Molekülen

ELISA

| M AB | Antigen IgA | IgG | IgM monoclonal(k) | BENCE JONES λ | BENCE JONES κ | FAB | FC |
|---|---|---|---|---|---|---|---|
| BS 50 Überstand | 0,609 | 0,607 | 0,111 | 0,452 | 0,128 | 0,538 | 0,170 |
| BS 50 ASCITES 1:4000 | 1,014 | 0,974 | 0,211 | 1,107 | 0,221 | 0,969 | 0,318 |
| BS 51 Überstand | 0,140 | 0,172 | 0,121 | 0,124 | 0,189 | 0,243 | 0,215 |
| BS 51 ASCITES 1:4000 | 0,284 | 0,291 | 0,376 | 0,235 | 0,212 | 0,345 | 0,303 |
| Anti-λ- ASCITES 1:4000 | 0,841 | 0,737 | 0,166 | 0,838 | 0,122 | 0,965 | 0,211 |
| Anti-κ- ASCITES 1:4000 | 0,679 | 0,778 | 0,672 | 0,157 | 0,376 | 0,863 | 0,209 |
| Kontrolle | 0,162 | 0,143 | 0,111 | 0,119 | 0,124 | 0,174 | 0,144 |

0133551

## Tabelle 4

### Immundadsorption von IL-2 an Antikörpersäulen aus BS 50

Angewandte IL-2-Aktivität       3840 Einheiten = 100 %

verbleibende IL-2-Aktivität
nach Immunadsorption mit BS 50       0 Einheiten =   0 %

verdünnte IL-2-Aktivität*       980 Einheiten =  27 %

* IL-2 war verdünnt mit
    0,5 M Propionsäure
    + 0,1 % PEG 6000
    + 0,1 % HSA

Patentansprüche

1. Hybridoma-Zell-Linie, die einen Antikörper mit Spezifität gegen Human-Interleukin-2 produziert, erhalten durch Fusion einer Antikörper produzierenden Tierzelle und einer neoplastischen Zelle, d a d u r c h g e k e n n z e i c h n e t, daß sie einen Antikörper produziert, der eine Struktur erkennt, die dem Human-Interleukin-2 (TCGF) und der leichten Kette λ von Humanimmunglobulin gemeinsam ist.

2. Zell-Linie nach Anspruch 1, dadurch gekennzeichnet, daß die Antikörper produzierende Zelle murinen Ursprungs ist.

3. Zell-Linie nach einem der vorstehenden Ansprüche, dadurch gekennzeichnet, daß die Antikörper produzierenden Zellen BALB/c Maus-Milzzellen sind.

4. Zell-Linie nach einem der vorstehenden Ansprüche, dadurch gekennzeichnet, daß die neoplastische Zelle eine Myeloma-Zelle ist.

5. Zell-Linie nach einem der vorstehenden Ansprüche, dadurch gekennzeichnet, daß die neoplastische Zelle die Myeloma-Zell-Linie P3-X63-Ag8.653 (C.N.C.M I-251) ist, die keine Antikörper produziert.

6. Hybridoma-Zell-Linie BS 50  C.N.C.M I-313

7. Monoklonaler Antikörper, dadurch gekennzeichnet, daß er eine Struktur erkennt, die dem Human-Interleukin-2 (TCGF) und der leichten Kette $\lambda$ von Humanimmunglobulin gemeinsam ist,und daß er von einer Hybridoma-Zell-Linie gemäß Anspruch 1 bis 6 produziert worden ist.

8. Verwendung des Antikörpers nach Anspruch 7 zur Bestimmung von Human-Interleukin-2 (TCGF) für diagnostische Zwecke und zur Verlaufskontrolle einer Therapie mit Interleukin-2-Präparaten.

9. Verwendung des Antikörpers nach Anspruch 7 zur Reinigung von Interleukin-2 (TCGF) für ein Therapieprodukt.

$MW \times 10^3$

66—

45—

26—

18—

14—

BJ: $\lambda$ $\kappa$

Fig. 1: SDS-Gel-Elektrophorese von BENCE JONES Protein $\lambda$ und Nachweis mit POD konjugiertem Antikörper aus BS 50 nach TRANS BLOT.

Fig. 2: Immunadsorption von IL-2 an Antikörpersäulen aus BS 50 im Vergleich zu anderen Antikörpersäulen nach Adsorption von leichter Kette λ

IL-2-haltiger Überstand

↓

1. Immundadsorption mit Anti- λ

| 2. Immunadsorption mit M AB | BS 51 | 3,143,8 | 5,186,17 | BS 50 | 6,85,21 | Kontrolle |
|---|---|---|---|---|---|---|
| verbleibende IL-2-Aktivität | 100% | 100% | 100 % | 0 % | 100% | 100 % |

0133551

0133551

Fig. 3

3/4

TAGE

Gesamtzellzahl (kumulativ)

$10^8$  $10^7$  $10^6$  $10^5$

5  10  15  20  25

0133551

Fig.3: (Fortsetzung)

Langzeitwuchs von MLC-Blasten mit IL-2 (TCGF),
welches von einer BS 50 Säule eluiert wurde.

o——o   Zusatz von 5% eluiertem Material zur Kultur
+——+   Zusatz von 2,5%      ''          ''          ''      ''

□——□ }
         Kontrollen mit 20 % Zusatz Lymphocult T $^R$
△——△ }

(Lymphocult T $^R$ : kommerzielles IL-2 Präparat der Fa. Biotest.)